Europäisches Patentamt

(19) European Patent Office

Office européen des brevets

(11) Numéro de publication: **0 064 419**
**B1**

(12) **FASCICULE DE BREVET EUROPEEN**

(45) Date de publication du fascicule du brevet:
**23.01.85**

(51) Int. Cl.⁴: **A 61 M 16/00**

(21) Numéro de dépôt: **82400474.1**

(22) Date de dépôt: **15.03.82**

(54) Tube endo-trachéal ou trachéotomique à ballon pour anesthésie.

(30) Priorité: **17.04.81 FR 8107820**

(43) Date de publication de la demande:
**10.11.82 Bulletin 82/45**

(45) Mention de la délivrance du brevet:
**23.01.85 Bulletin 85/4**

(84) Etats contractants désignés:
**BE CH DE FR GB IT LI LU**

(56) Documents cités:
**DE - A - 2 557 459**
**FR - A - 2 463 624**
**US - A - 4 327 721**
**US - A - 4 334 534**

**THE AMERICAN JOURNAL OF SURGERY, vol. 126,
octobre 1973, pages 529-533; S.STRONG et al.:" Laser
surgery in the aerodigestive tract".**

(73) Titulaire: **PORGES, Société Anonyme dite:, 9 à 15 rue
Léon Blum, F-91120 Palaiseau (FR)**

(72) Inventeur: **Milhaud, Alain, 11 Place Notre Dame,
F-80000 Amiens (FR)**

(74) Mandataire: **Bonnetat, Christian, Cabinet PROPI
Conseils 23 rue de Léningrad, F-75008 Paris (FR)**

## Description

L'invention concerne un tube endo-trachéal ou un tube trachéotomique à ballon pour anesthésie, notamment pour le traitement au laser de tissus pathologiques des voies respiratoires hautes.

On connaît déjà du document DE-A-2 557 459 un tube endo-trachéal pour anesthésie comportant un corps tubulaire servant à véhiculer le gaz de ventilation et d'anesthésie et pourvu au voisinage de son extrémité distale d'un ballon susceptible d'être gonflé depuis l'extérieur, par l'intermédiaire d'un canal auxiliaire, de manière à assurer l'étanchéité avec la trachée et à permettre ainsi une ventilation efficace. Un tel tube, ainsi que son ballon gonflable, est constitué de matières organiques naturelles ou de synthèse, afin de présenter une souplesse suffisante pour ne pas traumatiser les voies respiratoires dans lesquelles il est introduit.

Ce tube endo-trachéal connu donne toute satisfaction dans le cas d'une intervention classique. Toutefois, dans le cas d'un traitement au laser, qui présente des avantages certains par rapport aux techniques opératoires traditionnelles, des difficultés apparaissent.

En effet, la matière constitutive d'un tel tube endo-trachéal connu absorbe généralement bien l'énergie infrarouge, de sorte que si une anesthésie est délivrée par ce tube pendant une intervention au moyen d'un laser, on risque la détérioration, et même la destruction, dudit tube qui est destiné à délivrer les gaz assurant la respiration du patient. Il peut donc en résulter des conséquences graves pour celui-ci, d'autant plus que les matières organiques utilisées pour la fabrication des tubes endo-trachéaux sont non seulement combustibles, mais peuvent également dégager des vapeurs caustiques ou toxiques.

Il peut se faire que l'anesthésie ne puisse être pratiquée par les voies naturelles ou que, le patient étant déjà trachéotomisé, on n'utilise pas de tube endo-trachéal, mais un tube de trachéotomie portant un ballon d'anesthésie, ou que l'on remplace, si le malade est déjà trachéotomisé, la canule dont il est appareillé par une canule souple munie d'un ballon permettant l'anesthésie.

Les inconvénients d'utilisation du faisceau laser lorsque l'anesthésie est délivrée par tube trachéotomique, et non par tube endo-trachéal, sont bien entendu les mêmes.

Pour tenter à remédier à ces inconvénients, on a déjà pensé à protéger le tube endo-trachéal ou le tube trachéotomique au moyen d'enroulements hélicoïdaux d'une bande d'une matière réfléchissante de l'énergie infrarouge, telle que l'aluminium, ces enroulements étant par exemple fixés sur le tube au moyen d'un adhésif (cf notamment The American Journal of Surgery, Vol. 126 d'octobre 73, p. 533). Une telle solution peut être considérée comme acceptable pour la protection du tube lui-même, mais est sans effet pour protéger le ballon équipant le tube et qui a pour fonction d'assurer l'étanchéité et de permettre ainsi une ventilation efficace. Or, c'est justement le ballon qui est la partie dudit tube la plus vulnérable, car constitué d'une très faible épaisseur d'un élastomère sous tension.

On a également pensé à réaliser les tube endo-trachéaux en une matière métallique. Mais alors, on obtient des tubes rigides dont l'introduction est traumatisante pour les voies respiratoires. De plus, à cause du poli spéculaire d'un tel instrument, on risque des réflexions du faisceau laser sur des tissus sains ne devant pas être traités.

Afin de remédier à ces inconvénients, la Demanderesse a déjà décrit dans sa demande de brevet français Fr-A-2 463 624 un tube endo-trachéal ou trachéotomique pour anesthésie comportant un corps tubulaire servant à véhiculer les gaz de ventilation et d'anesthésie et pourvu au voisinage de son extrémité distale d'un ballon d'étanchéité susceptible d'être gonflé par l'intermédiaire d'un canal auxiliaire de gonflage et remarquable en ce qu'il comporte entre ledit ballon et l'extrémité proximale du tube, à quelque distance dudit ballon, un bouclier thermique souple.

Ainsi, grâce à ce bouclier thermique souple, il est possible d'obtenir un tube souple dans lequel le ballon, qui est la partie la plus fragile, est protégé par le bouclier thermique.

Un tel bouclier thermique peut se présenter sous la forme d'un disque coaxial au tube. Il peut être fixé sur celui-ci ou en être solidaire. Il peut être d'une seule pièce ou, au contraire, être constitué de plusieurs secteurs solidaires les uns des autres par des fentes dont les bords se chevauchent.

De préférence, le bouclier thermique est constitué d'une matière souple semblable à celle du tube et contenant une dispersion d'une poudre métallique finement divisée comme une poudre d'aluminium, d'argent, d'or, etc. . . Ces particules métalliques absorbent et réfléchissent l'énergie reçue de l'impact du faisceau laser servant au traitement des tissus pathologiques.

Seul le bouclier thermique peut contenir la dispersion de poudre métallique finement divisée. Cependant, il est avantageux que la totalité du tube comporte une telle dispersion métallique, de sorte que sa résistance à l'énergie infrarouge est améliorée.

Dans le cas d'un tube endo-trachéal, la distance séparant le ballon du bouclier est choisie approximativement égale à 2 ou 3 cm de façon à permettre le franchissement en deux temps des cordes vocales. Dans le cas d'un tube trachéotomique le problème du passage des cordes vocales ne se pose évidemment pas. Dans ces conditions, il est possible de placer le bouclier de protection thermique à quelques millimètres du ballon permettant l'étanchéité entre la trachée et le tube. Le diamètre du bouclier est choisi, dans les deux cas, légèrement inférieur au diamètre de la trachée à intuber.

Par ailleurs, on sait qu'en général, pendant le traitement au laser des tissus pathologiques des voies respiratoires hautes, on ventile le patient avec un mélange riche en oxygène. Aussi, l'éclatement intempestif du ballon supprimant l'étanchéité ou tout autre cause de fuite, permettrait alors au mélange gazeux contenu dans les poumons de refluer vers la partie haute de la trachée et d'être en contact avec le faisceau laser, ce qui pourrait entraîner des combustions intempestives du tube endo-trachéal et des muqueuses traitées par le laser.

Ainsi, malgré le bouclier de protection décrit ci-dessus, destiné à maintenir pendant toute la durée de l'intervention l'intégrité du ballon trachéal assurant l'étanchéité ventilatoire et interdisant le reflux de l'oxygène, un danger subsiste que, soit par défaut d'étanchéité du ballon, soit en raison de son éclatement, la partie haute de la trachée puisse contenir un mélange gazeux anormalement enrichi en oxygène.

De plus, il faut considérer que l'air contenant 20% d'oxygène constitue déjà un comburant actif. Il est donc nécessaire que les tirs laser soient pratiqués dans une atmosphère appauvrie en oxygène.

La présente invention a pour objet un tube endo-trachéal ou trachéotomique pour anesthésie permettant d'éviter les inconvénients mentionnés ci-dessus, dûs à un manque d'étanchéité ou à un éclatement du ballon.

A cette fin, selon l'invention, le tube endo-trachéal ou trachéotomique pour anesthésie comportant un corps tubulaire servant à véhiculer les gaz de ventilation et d'anesthésie et pourvu au voisinage de son extrémité distale d'un ballon d'étanchéité susceptible d'être gonflé par l'intermédiaire d'un canal auxiliaire de gonflage (2), un bouclier thermique souple étant disposé entre ledit ballon et l'extrémité proximale du corps tubulaire, est remarquable en ce qu'il comporte un autre canal auxiliaire débouchant du côté dudit bouclier opposé au ballon et destiné à permettre en cours d'utilisation l'oppravisionnement en gaz inerte de l'espace situé dudit côté du bouclier.

Ainsi, grâce à un canal auxiliaire, on peut effectuer le balayage permanent de la partie haute de la trachée (constituant en quelque sorte le champ opératoire) au moyen d'un courant d'azote, par exemple. Le gaz inerte remplit ainsi toute la partie haute de la trachée.

Dans ces conditions, les risques d'inflammation sont pratiquement supprimés.

Par ailleurs, on sait que l'instrument permettant le tir laser est encombrant et que l'intubation nasale, en général, oblige à placer le tube endo-trachéal dans des dispositions, telles que des obturations intempestives du canal de ventilation du tube puissent se présenter.

Aussi, de préférence, le tube selon l'invention comporte une armature hélicoïdale métallique ou un fil synthétique polyamide polyester, par exemple, interdisant l'écrasement des parois l'une sur l'autre, et donc, leur obturation.

Les figures du dessin annexé feront bien comprendre comment l'invention peut être réalisée.

La fig. 1 est une vue en perspective d'un tube endo-trachéal selon l'invention.

La fig. 2 est une vue de face agrandie d'une variante de réalisation du bouclier thermique.

La fig. 3 est une coupe selon la ligne III-III de la fig. 2.

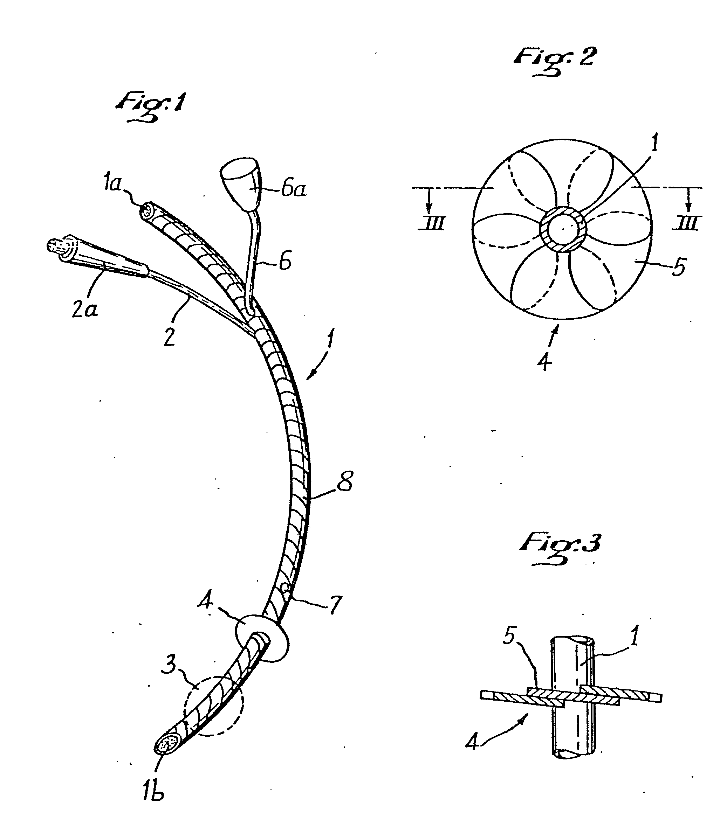

Le tube endo-trachéal ou trachéotomique, montré par la fig. 1, comporte de façon connue un corps tubulaire 1 servant à véhiculer les gaz de ventilation et d'anesthésie depuis son extrémité proximale 1a jusqu'à son extrémité distale 1b et un canal auxiliaire 2 pourvu d'une canule 2a à son extrémité proximale et s'incorporant au corps tubulaire 1. Le canal 2 sert au gonflage d'un ballon 3, apte à assurer l'étanchéité avec les tissus de la trachée.

Par ailleurs, le corps tubulaire 1 comporte, en avant zu ballon gonflable 3, un bouclier thermique souple 4.

Sur la fig. 1, ce bouclier thermique 4 est formé par un disque solidaire dudit corps tubulaire, orthogonalement à l'axe de celui-ci.

En revanche, sur les fig. 2 et 3, le mode de réalisation du bouclier thermique représenté est constitué de plusieurs secteurs 5 solidaires à leur base du corps tubulaire 1 et se recouvrant partiellement, à la manière de pétales. Les secteurs 5 se déforment facilement lors du franchissement de passages étroites, mais retrouvent élastiquement leur position naturelle lorsque le tube est en place dans la trachée.

De plus, le tube endo-trachéal ou trachéotomique 1 comporte un autre canal auxiliaire 6, pourvu d'une canule 6a à son extrémité proximale et s'incorporant au corps tubulaire 1 pour déboucher, à son extrémité distale, par un orifice 7 de la paroi du corps tubulaire 1. L'orifice 7 se trouve du côté du bouclier 4 opposé au ballon 3.

Le corps tubulaire 1 et ses différents éléments 2, 3, 4 et 6 peuvent être fabriqués dans différentes matières : les élastomères naturels, les polyisoprènes de synthèse, les polychloroprènes, les polybutadiènes-styrènes; les polybutadiènes, soit purs, soit en combinaison peuvent éventuellement convenir. Il en est de même des chlorures de polyvinyle plastifiés, des polyuréthanes ..., mais il est préférable d'utiliser des polysiloxanes, tout particulièrement résistant à l'élévation de température et dont la dégradation thermique n'entraîne pas la formation de vapeurs caustiques ou toxiques.

En pratique, il suffit pour protéger le ballon 3 contre le laser, que le tube en silicone soit équipé au-dessus du ballon, d'un bouclier 4 de diamètre légèrement inférieur au diamètre de la trachée à intuber et d'une épaisseur d'environ 6 à 8/10 de mm, qui est par exemple fabriqué en silicone chargé en poudre d'aluminium.

Des essais expérimentaux ont montré qu'une proportion de 1% d'aluminium en poids permet de garantir une résistance à 100 impulsions de 5/10 de seconde au rayon laser.

De plus, afin d'éviter l'écrasement du corps tubulaire 1 lorsque celui-ci est plié, il est avantageux de prévoir un revêtement en hélice 8, constitué d'une bande métallique ou de fibres synthétiques, par exemple.

En utilisation, le ballon 3 est gonflé et prend appui sur les parois de la trachée (non représentées) du patient, le gonflage étant obtenu par l'envoi d'un gaz sous pression à travers le canal 2. Par suite, le ballon 3 assure la séparation entre l'extrémité distale 1b du corps tubulaire 1 et les parties du tube disposées au-dessus du ballon 3. Ainsi, l'oxygène envoyé dans le tube par son extrémité 1a peut ventiler les poumons du patient. De même, par le conduit 6 on envoie un courant d'azote qui ressort à travers l'orifice 7. Le ballon 3 sépare donc deux domaines où règnent des ambiances gazeuses différentes : au-dessous du ballon 3 de l'oxygène sort à travers l'orifice 1b, alors qu'au-dessus du ballon 3 de l'azote sort par l'orifice 7.

Le traitement au laser s'effectue donc dans une ambiance d'azote. Dans le cas où se produirait une mise en communication des domaines séparés par le ballon 3, le balayage d'azote sortant par l'orifice 7 empêcherait l'oxygène de ventilation des poumons du patient de s'enflammer ou d'exploser sous l'action du faisceau laser.

## Revendications

1. Tube endo-trachéal ou trachéotomique pour anesthésie comportant un corps tubulaire (1) servant à véhiculer les gaz de ventilation et d'anesthésie et pourvu au voisinage de son extrémité distale (1b) d'un ballon d'étanchéité (3) susceptible d'être gonflé, par l'intermédiaire d'un canal auxiliaire de gonflage (2) un bouclier thermique souple (4) étant disposé entre ledit ballon (3) et l'extrémité proximale (1a) du corps tubulaire (1) caractérisé en ce qu'il comporte un autre canal auxiliaire (6) débouchant (en 7) du côté dudit bouclier (4) opposé au ballon (3) et destiné à permettre en cours d'utilisation l'approvisionnement en gaz inerte de l'espace situé dudit côté du bouclier.

2. Tube selon la revendication 1, caractérisé en ce que ledit corps tubulaire (1) est pourvu d'un revêtement (8) évitant son écrasement lorsqu'il est plié.

## Patentansprüche

1. Endotracheal- oder Tracheotomröhre zur Verwendung bei der Narkose und bestehend aus einem Röhrenkörper (1), der der Weiterleitung des Belüftungs- und Anästhesiegases dient und am distalen Ende (1b) einen Dichtungsballon (3), der mittels eines Hilfskanals (2) aufblasbar ist, und ein geschmeidiges Wärmeschild (4) aufweist, das zwischen dem Ballon (3) und dem näher gelegenen Ende (1a) des Röhrenkörpers liegt, gekennzeichnet durch einen weiteren Hilfskanal (6), der (bei 7) von der dem Ballon gegenüberliegenden Seite des Schildes (4) austritt und dazu dient, von dem auf der Seite des Schildes gelegenen Raum während der Verwendung Inertgas einzuspeisen.

2. Röhre nach Anspruch 1, dadurch gekennzeichnet, daß der Röhrenkörper (1) mit einer Umkleidung (8) versehen ist, wodurch vermieden wird, daß er beim Biegen oder Falten gequetscht wird.

## Claims

1. An endo-tracheal or tracheotomic tube for anesthesia comprising a tubular body (1) for conveying the ventilation and anesthesia gases and provided in the vicinity of its distal end (1b) with a ballon (3) ensuring tightness and inflatable through an auxiliary inflation conduit (2), a flexible thermal shield being disposed between said balloon (3) and the proximal end (1a) of the tubular body (1), characterized in that it comprises a further auxiliary conduit (6) opening (at 7) on the side of said shield (4) opposite the balloon (3) and adapted to supply the space located on said side of the shield with an inert gaz, during use.

2. The tube of Claim 1, characterized in that said tubular body (1) provided with a coating (8) which avoids crushing thereof when it is bent.

*Fig:1*

*Fig:2*

*Fig:3*